# EUROPEAN PATENT SPECIFICATION

(11) **EP 0 648 512 B1**
(45) Date of publication and mention of the grant of the patent: **26.05.1999**
(21) Application number: 94203021.4
(22) Date of filing: 18.10.1994
(51) Int. Cl.: A61M 25/02

(54) **Bracket for connecting device with the interior of the human body**
Bügel für eine Verbindungsvorrichtung mit dem Inneren des menschlichen Körpers
Bride pour dispositif de connexion avec l'intérieur du corps humain

(30) Priority: 19.10.1993 NL 9301810
(43) Date of publication of application: 19.04.1995
(73) Proprietor: N.V. Nutricia, NL-2712 HM Zoetermeer (NL)
(72) Inventor: Van Heesch, Theodorus Ido Wilhelmus, NL-2611 GK Delft (NL)
(74) Representative: Hoijtink, Reinoud

(56) References cited:
- EP-A- 0 327 299
- WO-A-93/17738
- WO-A-94/08648
- FR-A- 2 023 057
- GB-A- 2 147 811
- US-A- 4 397 647

## Description

The invention relates to a bracket for securing a flexible hose protruding from the interior of an organ of the human body, in particular the stomach, through an opening in the wall to a location outside the body, said bracket comprises a clamping part clampingly receiving the hose, a bend piece connecting through roughly a right angle to the clamping part, and a fixing part intended for placing on the skin, in which said clamping part runs roughly parallel to said fixing part and consists of successive flexible cylindrical portions.

Such a bracket is used for medical purposes, particularly for patients who are not capable of taking in food in the normal manner. In order to introduce food directly into the stomach a flexible hose is introduced via the oesophagus, which hose is carried to the outside via an orifice in the stomach wall and in the tissue. On the end part of the inserted hose a plate-like holding member is arranged to hold the hose inside the stomach in operationally reliable manner. The other end part of the hose is secured in a bracket.

GB-A-2147811 describes a bracket for anchoring catheter tubing to the skin of a patient. The bracket includes an arcuate passageway for receiving and redirecting the tubing. The passageway is externally accessible along its length by manually spreading the two halves of the bracket along a slit. The slit is held closed by means of a cable tie which is tightened in an annular channel grooved into the circumference of the bracket whereby the tubing is prevented from sliding through or from the passageway. In order to enable replacement or cleaning of the tubing and the bracket the tie must be severed to permit removal of the tubing from the passageway. Severing the tie is difficult as the walls of the channel are obstructing the passageway of severing means in order to be placed under the cable tie. Applying this known bracket is also difficult because at the same moment the two halves of the bracket has to be held closed, the cable tie has to be applied into the channel and the free end of the tie must be pulled while the tie has to be prevented from slipping in the channel.

The invention has for its object to obviate these drawbacks in a simple manner.

This is achieved according to the invention in that a gap is provided in the wall of each cylindrical portion, said gaps extending parallel to the clamping part and being arranged substantially diametrically opposite each other in said successive cylindrical portions.

The bracket according to the invention can be easily applied and removed. On the one hand the flexible hose is safely secured by pulling it into the gap without the necessity of additional clamping means. Because the gap is arranged shifted through roughly 180° in successive cylindrical portions the flexible hose remains secured to the bracket if a force in any direction is applied to the hose. On the other hand the bracket can be easily removed from or even be pushed over the hose by nursing staff for the purpose of, for example, cleaning without the necessity of using additional means.

The invention is further elucidated with reference to the drawing of an embodiment.

In the drawing:
Fig. 1 shows a perspective view of a device according to the invention arranged on the human body,
Fig. 2 shows a perspective view of the device according to the invention,
Fig. 3 shows in perspective the clamping bracket according to the invention, and
Fig. 4 shows a detail of the placing of the device according to the invention.

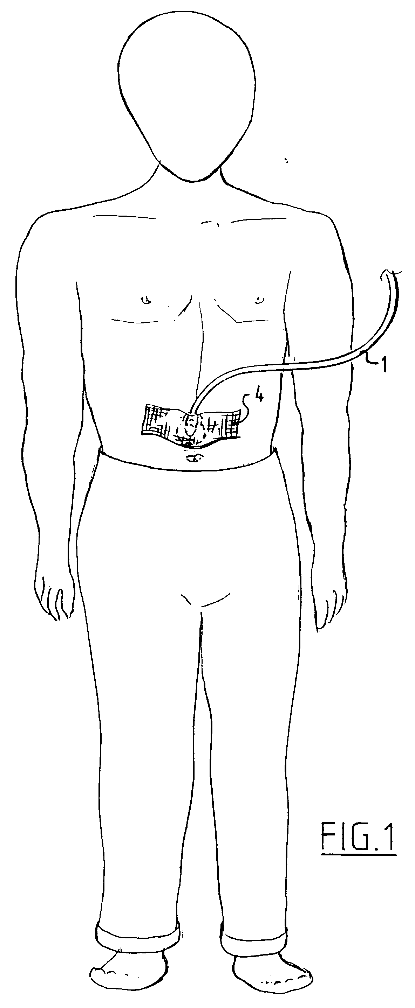

In a particular category of patients an opening is made as according to fig. 1 in the stomach and abdominal wall to enable artificial administering of food via a flexible hose.

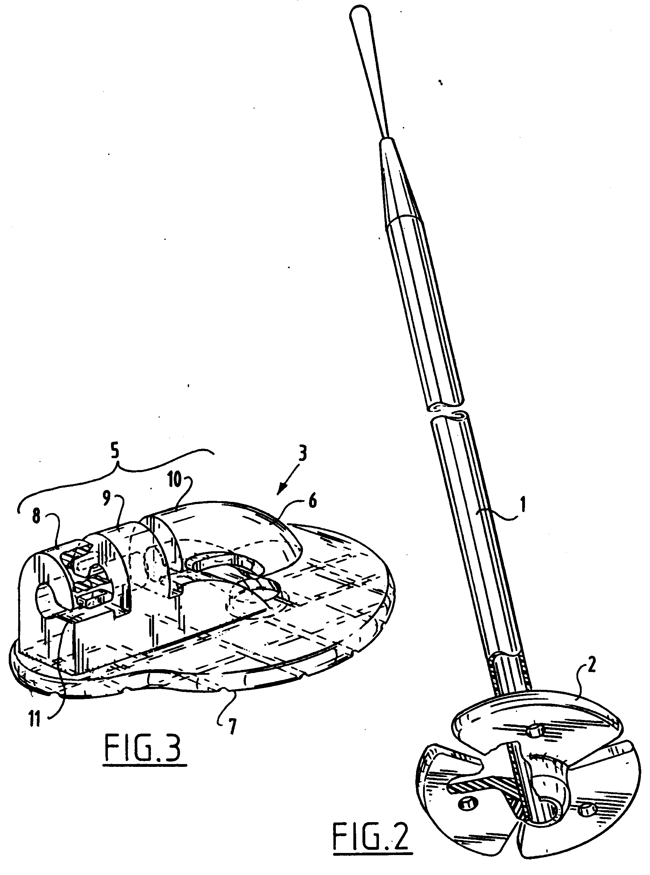

For this purpose is used a flexible hose 1, a holding member of flexible material arrangeable on the end of the hose and intended to fixedly hold hose 1 on the inside of the stomach wall. The hose 1 and the holding member 2 are introduced via the oesophagus. Arranged on the other end part of the hose is a clamping bracket 3 as according to fig. 3 intended for securing to the abdominal wall of the patient with for instance a plaster 4 (see fig. 1). The clamping bracket consists of a clamping part 5 and a bend piece 6. The clamping part 5 and the bend piece 6 are placed on a fixing part 7. The whole bracket is of flexible skincompatible material and distributes the force exerted by the hose uniformly round the wound. The fixing part 7 is secured to the abdominal wall using a plaster 4. The clamping part 5 runs parallel to the fixing part 7 and is formed by successive flexible cylindrical portions 8, 9, 10. The cylindrical portions each have a longitudinal gap, for instance 11. The longitudinal gaps for successive cylindrical portions are shifted through 180° relative to each other.

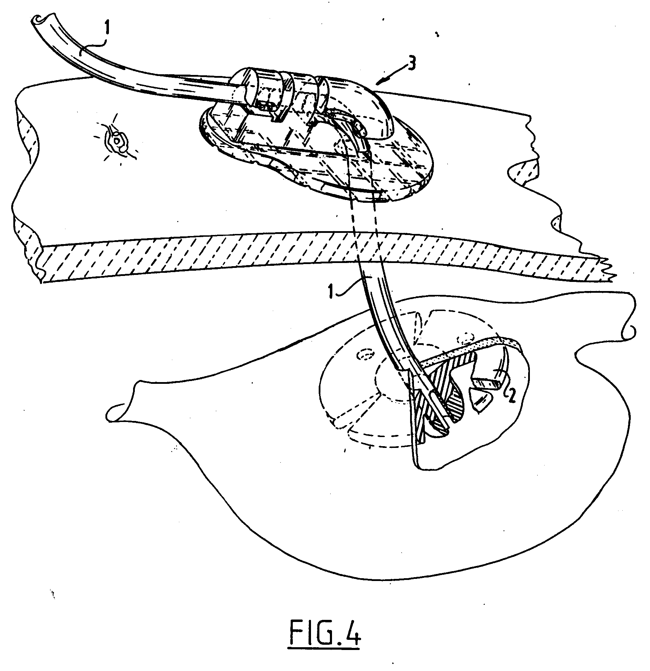

The hose can be bent through 90° directly upon leaving the body and then runs practically parallel to the abdominal wall of the patient (see fig. 4). For the purpose of cleaning, the clamping bracket 3 can be released from the abdominal wall by nursing staff and pushed over the hose or removed therefrom. After cleaning of the bracket and the hose part the bracket can be replaced using a plaster.

## Claims

1. Bracket (3) for securing a flexible hose (1) protruding from the interior of an organ of the human body, in particular the stomach, through an opening in the wall to a location outside the body, said bracket (3) comprises a clamping part (5) clampingly receiving the hose, a bend piece (6) connecting through roughly a right angle to the clamping part (5), and a fixing part (7) intended for placing on the skin, in which said clamping part (5) runs roughly parallel to said fixing part (7) and consists of successive flexible cylindrical portions (8, 9, 10), **characterized in that** a gap is provided in the wall of each cylindrical portion, said gaps extending parallel to the clamping part and being arranged substantially diametrically opposite each other in said successive cylindrical portions.

2. Apparatus comprising a flexible hose (1) and a holding member (2) placeable on an end part of the hose and a bracket (3) as claimed in claim 1.

## Patentansprüche

1. Klammer (3) zur Festlegung eines flexiblen Schlauches (1), der aus dem Inneren eines Organs des menschlichen Körpers, insbesondere aus dem Magen, über eine Öffnung in der Bauchdecke nach einer Stelle außerhalb des Körpers vorsteht, wobei die Klammer (3) einen Klemmkörper (5) zum Festklemmen des Schlauches und ein Kniestück (6) aufweist, das den Schlauch um etwa einen rechten Winkel mit dem Klemmkörper (5) verbindet und wobei eine Festlegungseinrichtung (7) vorgesehen ist, die auf der Haut festgelegt werden soll und in der der Klemmkörper (5) etwa parallel zu der Festlegeeinrichtung (7) verläuft und der Klemmkörper aus aufeinanderfolgenden flexiblen zylindrischen Abschnitten (8, 9, 10) besteht,
dadurch gekennzeichnet, daß in der Wand eines jeden zylindrischen Teils ein Spalt angeordnet ist, der parallel zum Klemmkörper verläuft, wobei benachbarte Spalte etwa diametral gegeneinander in den aufeinanderfolgenden zylindrischen Abschnitten versetzt sind.

2. Vorrichtung mit einem flexiblen Schlauch (1) und einem Haltekörper (2), der an einem Ende des Schlauches festgelegt ist, und mit einer Klammer (3) gemäß Anspruch 1.

## Revendications

1. Support (3) pour fixer un tuyau flexible (1) faisant saillie de l'intérieur d'un organe du corps humain, en particulier l'estomac, à travers une ouverture dans la paroi jusqu'à un emplacement à l'extérieur du corps, ledit support (3) comprend une pièce de serrage (5) recevant par serrage le tuyau, une pièce cintrée (6) étant raccordée au moyen d'un angle à peu près droit à la pièce de serrage (5), et une pièce de fixation (7) prévue pour être placée sur la peau, dans lequel ladite pièce de serrage (5) s'étend à peu près parallèlement à ladite pièce de fixation (7) et se compose de parties cylindriques flexibles (8, 9, 10) successives, caractérisé en ce qu'un espace est prévu dans la paroi de chaque partie cylindrique, lesdits espaces s'étendant parallèlement à la pièce de serrage et étant agencés sensiblement diamétralement opposés les uns aux autres dans lesdites parties cylindriques successives.

2. Appareil comprenant un tuyau flexible (1) et un organe de maintien (2) pouvant être placés sur une partie d'extrémité du tuyau et d'un support (3) selon la revendication 1.
